# EUROPEAN PATENT APPLICATION

(11) **EP 2 457 899 A1**
(43) Date of publication of application: **30.05.2012**
(21) Application number: 10802199.9
(22) Date of filing: 07.07.2010
(51) Int. Cl.: C07D 211/60, C07B 57/00

(54) **PROCESS FOR PRODUCTION OF OPTICALLY ACTIVE NIPECOTAMIDE**

(30) Priority: 21.07.2009 JP 2009170487
(71) Applicant: Sumitomo Chemical Company, Limited, Tokyo 104-8260 (JP)
(72) Inventor: WATANABE, Yosuke, Toyonaka-shi Osaka 560-0021 (JP); OHTANI, Yutaka, Kobe-shi Hyogo 657-0036 (JP)
(74) Representative: Nieuwenhuys, William Francis
(86) International application number: PCT/JP2010/061858
(87) International publication number: WO 2011/010579

(57) **Abstract**

Optically active nipecotamide can be produced by a method for producing optically active nipecotamide comprising: a step of reacting nipecotamide with optically active lactic acid to prepare a mixture of diastereomer salts and then allowing one diastereomer salt in the mixture of the diastereomer salts to precipitate; a step of collecting the precipitated diastereomer salt; and, a step of treating the collected diastereomer salt with a base to cause optically active nipecotamide to release.

## Description

### TECHNICAL FIELD

The present invention relates to a method for producing an optically active nipecotamide by optical resolution.

### BACKGROUND ART

Nipecotamide (piperidine-3-carboxamide), in particular, an optically active nipecotamide is known to be a useful compound as a material for producing pharmaceutical products. A method for producing an optically active nipecotamide is known, where R-nipecotamide is obtained from nipecotamide using an enzyme which is able to hydrolyze S-nipecotamide selectively (see WO 2008/102720).

### SUMMARY OF THE INVENTION

However, the method described in WO 2008/102720 has problems, for example, rather a high cost of the enzyme used, unavailability of the enzyme and need for special equipments such as a centrifuge separator to remove solids including the enzyme after hydrolysis. Thus, in view of commercial production, the method has not been satisfactory.

The present invention allows to obtain an optically active nipecotamide with a simple operation and with efficiency by conducting optical resolution using an optically active lactic acid.

That is to say, the present invention provides a method for producing an optically active nipecotamide, the method comprising: a step of reacting nipecotamide with an optically active lactic acid in a solvent to prepare a diastereomer salt mixture and then allowing one of the diastereomer salts contained in the diastereomer salt mixture to precipitate; a step of collecting the precipitated diastereomer salt; and, a step of treating the collected diastereomer salt with a base to liberate an optically active nipecotamide.

The present invention further provides a diastereomer salt mixture of nipecotamide and an optically active lactic acid.

### MODES FOR CARRYING OUT THE INVENTION

Nipecotamide used in the production method of the present invention is a mixture of R-nipecotamide and S-nipecotamide, and usually a racemic form is used. Nipecotamide is commercially available, and a commercial product may be used as is. Nipecotamide obtained by reducing nicotinamide also may be used.

Reduction of a nicotinamide can be conducted, for example, by catalytic reduction in a solvent in the presence of a catalyst. The solvent may be, for example, a C₁ to C₆ alcohol such as methanol, ethanol, propyl alcohol and butyl alcohol; an ether such as tetrahydrofuran; acetic acid; water; and a mixed solvent thereof, and among them, propyl alcohol (particularly, 2-propanol) is preferable. The amount of the solvent used is preferably within the range of from 3 to 5 mL relative to 1 g of nicotinamide. Examples of the reduction catalyst include a palladium catalyst and a platinum catalyst, preferably a palladium catalyst, particularly a palladium catalyst supported on carbon. The amount of the reduction catalyst used is preferably within the range of from 0.005 to 0.02 parts by weight, in terms of parts by weight of the metal in the reduction catalyst, relative to 1 part by weight of nicotinamide.

The temperature of the catalytic reduction is preferably within the range of from 70 to 80°C. The hydrogen pressure of the catalytic reduction is preferably within the range of from 0.1 to 1 MPa.

Nipecotamide obtained by the catalytic reduction is usually in a racemic form.

The present invention is one obtaining an optically active nipecotamide by optically resolving nipecotamide, and a method for producing an optically active nipecotamide, the method comprising: a step of reacting nipecotamide with an optically active lactic acid in a solvent to prepare a diastereomer salt mixture and then allowing one of the diastereomer salts contained in the diastereomer salt mixture to precipitate; a step of collecting the precipitated diastereomer salt; and, a step of treating the collected diastereomer salt with a base to liberate an optically active nipecotamide.

An optically active lactic acid is commercially available, and a commercial product may be used as is. In order to enhance the optical purity of the obtained optically active nipecotamide, it is favorable to use an optically active lactic acid with high optical purity. The optical purity of an optically active lactic acid is preferably 90 % ee or more, more preferably 95 % ee or more, much more preferably 98 % ee or more, and especially preferably 98.5 % ee or more.

The amount of the optically active lactic acid used is preferably in a ratio of from 0.5 to 1. 5 mol, and more preferably from 0.8 to 1.2 mol, relative to 1 mol of nicotinamide.

In the step of reacting nipecotamide with an optically active lactic acid in a solvent to prepare a diastereomer salt mixture and then allowing one of the diastereomer salts contained in the diastereomer salt mixture to precipitate, the order of mixing a solvent, nipecotamide and an optically active lactic acid is not particularly limited, but preferably, a nipecotamide solution is prospectively prepared from a solvent and nipecotamide at first, and followed by adding an optically active lactic acid to the nipecotamide solution. In preparing the nipecotamide solution, the solvent may be appropriately heated. Alternatively, an optically active lactic acid solution, for example an aqueous solution, may be prepared by dissolving an optically active lactic acid in an adequate solvent, followed by adding the optically active lactic acid solution to a nipecotamide solution.

When an aqueous solution of the optically active lactic acid is added, it is mixed with the nipecotamide solution to obtain a mixture, and then, the resulting mixture can also be, for example, concentrated or dehydrated to promote the precipitation of a diastereomer salt. The concentration of the aqueous solution of the optically active lactic acid is not particularly limited, but preferably, it is 85 % by weight or more.

Adding the optically active lactic acid is preferably conducted little by little. The adding temperature of the optically active lactic acid is equal to or less than the boiling point of the solvent used, preferably in the range of from 0 to 100°C.

The solvent used to react nipecotamide with an optically active lactic acid includes an alcohol solvent, a ketone solvent, an ester solvent, an ether solvent, a sulfur-containing solvent, a nitrogen-containing solvent, a lactone solvent and water. These solvents may be used alone, or may be used in a mixture of two or more.

Specific examples of the alcohol solvent are C₁ to C₆ alcohols such as methanol, ethanol, propyl alcohol and butyl alcohol, and among them, butyl alcohol, particularly 1-butanol, is preferable.

Specific examples of the ketone solvent are acetone, methyl ethyl ketone and methyl isobutyl ketone, and among them, methyl isobutyl ketone is preferable.

Specific examples of the ester solvent are acetate esters such as ethyl acetate, propyl acetate and butyl acetate.

Specific examples of the ether solvent are methyl t-butyl ether, tetrahydrofuran and dioxane.

Specific examples of the sulfur-containing solvent are dimethyl sulfoxide and sulfolane.

Specific examples of the nitrogen-containing solvent are pyrrolidone and dimethylformamide.

A specific example of the lactone solvent is γ-butyrolactone.

Above all, an alcohol solvent, for example, 1-butanol is preferable. Moreover, by adding a ketone solvent or an ester solvent to an alcohol solvent, the solubility of a target diastereomer salt can be reduced to improve the yield. Thus, a mixed solvent of an alcohol (for example, 1-butanol) and a ketone (for example, methyl isobutyl ketone), or a mixed solvent of an alcohol (for example, 1-butanol) and an ester (for example, ethyl acetate) is also preferable. In the mixed solvent of an alcohol and a ketone, the mixing ratio in terms of volume is preferably in the range of 1 : 0.01 to 1 : 1.2. In the mixed solvent of an alcohol and an ester, the mixing ratio in terms of volume is preferably in the range of 1 : 0.01 to 1 : 1.2.

Water may be further added to these solvents. When a solvent containing water is used, the optical yield of a diastereomer salt obtained by fractional crystallization and the optical purity of an optically active nipecotamide obtained from the diastereomer salt may be improved.

The amount of the solvent used is as much as necessary to dissolve nipecotamide, and is usually in the ratio of 1 to 50 parts by weight, preferably 4 to 20 parts by weight, relative to 1 part by weight of nipecotamide. When water is contained in the solvent, water is preferably used in an amount of 0.07 to 0.15 parts by weight relative to 1 part by weight of nipecotamide.

When D-lactic acid is used as the optically active lactic acid, the diastereomer salt mixture obtained is a mixture of two kinds of diastereomer salts of D-lactic acid salt of S-nipecotamide and D-lactic acid salt of R-nipecotamide. When L-lactic acid is used as the optically active lactic acid, the diastereomer salt mixture obtained is a mixture of two kinds of diastereomer salts of L-lactic acid salt of S-nipecotamide and L-lactic acid salt of R-nipecotamide.

In order to precipitate the mixture of diastereomer salts, the solvent may be cooled. For example, a solvent containing nipecotamide and an optically active lactic acid, which is heated to 50 to 80°C, is cooled to a temperature in the range of from -10 to 35°C, preferably in the range of from 0 to 30°C, thereby allowing one of the diastereomer salts to be precipitated. In this case, the cooling is preferably conducted in a gradual manner in view of the chemical purity and the optical purity of the optically active nipecotamide finally obtained. A target diastereomer salt may be prepared in advance and be used as a seed crystal.

The step of collecting the precipitated diastereomer salt is conducted in a usual solid-liquid separation procedure.

Specifically, separation operation such as filtration and decantation is recited.

As a result, a high purity diastereomer salt, i.e., a diastereomer salt with little contamination of the other diastereomer salt, can be obtained. The purity of the resulting diastereomer salt can be even more enhanced by conducting a purifying operation such as washing with a solvent, recrystallization and column chromatography on the diastereomer salt.

When D-lactic acid is used as the optically active lactic acid, D-lactic acid salt of R-nipecotamide is usually obtained, whereas when L-lactic acid is used as the optically active lactic acid, L-lactic acid salt of S-nipecotamide is usually obtained.

Particularly, when an alcohol solvent (especially, 1-butanol), a mixed solvent of an alcohol (especially, 1-butanol) and a ketone (especially, methyl isobutyl ketone), and a mixed solvent of an alcohol (especially, 1-butanol) and an ester (especially, ethyl acetate) are used, the fractional crystallization of D-lactic acid salt of R-nipecotamide, or L-lactic acid salt of S-nipecotamide becomes advantageous.

In this manner, an optically active lactic acid salt of an optically active nipecotamide can be obtained in a solid state. When the solvent contains water, the optically active lactic acid may take the form of a hydrate.

The liquid phase after the precipitated one of the diastereomer salts is collected contains much of the other diastereomer salt. Therefore, the other diastereomer salt can be removed by a process such as cocentrating the liquid phase, crystallizing the diastereomer salt by adding another solvent and the live.

Then, the collected diastereomer salt is treated with a base to liberate an optically active nipecotamide, and thus the optically active nipecotamide is produced.

The diastereomer salt obtained in the previous step has formed an optically active lactic acid and a salt, and an optically active nipecotamide can be liberated by treating with a base.

Specific examples of the base used are an alkali metal hydroxide such as sodium hydroxide and potassium hydroxide; an alkali metal carbonate such as sodium carbonate and potassium carbonate; an alkali metal hydrogen carbonate such as sodium hydrogen carbonate and potassium hydrogen carbonate; an alkali metal alkoxide such as sodium methoxide, sodium ethoxide, potassium methoxide and potassium ethoxide; and a tertiary amine such as triethylamine, trimethylamine and diisopropylethylamine.

The amount of the base used is preferably in the ratio of 1 to 3 mol relative to 1 mol of diastereomer salt.

The base treatment of the diastereomer salt is usually conducted by mixing the diastereomer salt with the base in a solvent. The solvent may be an alcohol such as butyl alcohol, an ether such as tetrahydrofuran, water and a mixed solvent thereof. The amount of the solvent used is preferably in the ratio of 3 to 20 mL relative to 1 g of diastereomer salt. The mixing temperature is preferably in the range of from 0 to 50°C, and the time for mixing is preferably in the range of from 1 minute to 24 hours.

The reaction mixture after treating the diastereomer salt with a base contains an optically active lactic acid and an optically active free nipecotamide, and the optically active lactic acid may form a salt with the base used. An optically active nipecotamide may be isolated from the reaction mixture by a usual operation such as extraction, filtration, concentration and crystallization.

A typical operation for treating a diastereomer salt with a base is as follows: A diastereomer salt is mixed with water to make an aqueous solution or an aqueous dispersion, to which a base is added to make it basic. In the process, the pH of the solution or the dispersion is preferably 10 or greater. After the temperature of the solution or the dispersion is kept at 20 to 30°C, an organic solvent which is capable of phase separation with water is added and stirred. Then, an optically active nipecotamide is extracted in the organic layer, and a salt of an optically active lactic acid and the base is extracted in the aqueous layer. The mixture is then left still until the organic layer and the aqueous layer are separated sufficiently. Then the organic layer is removed by liquid separation, and an optically active nipecotamide can be obtained from the organic layer.

The organic solvent includes, for example, a C₄ to C₆ alcohol such as butyl alcohol and pentyl alcohol, an ester such as ethyl acetate and methyl acetate, and an ether such as tetrahydrofuran and methyltetrahydrofuran. The amount of the organic solvent used is preferably in the ratio of 3 to 20 mL relative to 1 g of diastereomer salt.

If necessary, the organic layer may be washed with water. Extraction operation can be conducted again on the aqueous layer separated by the liquid separation stated above in order to improve the yield of the optically active nipecotamide. The organic solvent may be distilled off from the organic layer thus obtained, and an optically active nipecotamide can be isolated.

The isolated optically active nipecotamide may be further purified by a treatment operation such as recrystallization and column chromatography. Alternatively, an optically active nipecotamide may be dissolved in an adequate solvent, and then isolated as an acid addition salt by adding a desired acid.

From the aqueous layer after the extraction operation, an optically active lactic acid may be recovered by an ordinary method. The recovered optically active lactic acid may be subjected, as is or after being purified, to the next step, in which case the optically active lactic acid can be reused.

### EXAMPLES

Hereinafter, the present invention is described in more detail by the following examples, however, the present invention is by no means limited to the examples.

The optical purity of the optically active nipecotamide obtained was determined by the following process: From the diastereomer salt obtained in each of the examples, an optically active nipecotamide, which was liberated by triethylamine, was isolated, and was converted into a derivative by 3,5-dinitrobenzoyl chloride, and then, the optical purity was determined by the area normalization method using a high-performance liquid chromatography. The analytic conditions were as follows:

### <Analytic Conditions>

Column: CHIRALCEL OD-RH (from DAICEL CORPORATION), 4.6 mm × 150 mm (Column Temperature 40°C)
Mobile Phase: A = Water, B = Acetonitrile

**Table 1**

| Time (minutes) | 0 | 10 | 15 | 15.01 | 30 |
|---|---|---|---|---|---|
| Concentration A | 90% | 90% | 70% | 50% | 10% |
| Concentration B | 10% | 10% | 30% | 50% | 90% |

Flow Rate: 1.0 mL/min.
Detection: UV 230 nm
Retention Time: S-nipecotamide = 19.8 min., R-nipecotamide = 20.1 min.

### <Production of Nipecotamide>

122.12 g (1.00 mol) of nicotinamide was dissolved in 500 mL of 2-propanol. To the resulting solution was charged 14.4 g of palladium-carbon (10 %), and the solution was stirred for 4 hours at 75°C under hydrogen pressure of 0.5 MPa. After the reaction was completed, the palladium-carbon was filtered and separated. The filtered palladium-carbon was washed with 100 mL of 2-propanol. The filtrate and the washing liquid were combined and condensed, and 126.08 g of white crystals of nipecotamide in a racemic form was obtained. The yield was 98.4 %. The resultant was confirmed to be the target object by NMR.

### Example 1 Optical Resolution of Nipecotamide

Ten grams (10g, 78.0 mmol) of nipecotamide was dissolved in a mixed solvent of 30 mL of 1-butanol and 30 mL of ethyl acetate. To the solution was added 8.53 g (98.6 % ee, 90 % aqueous solution, 85.8 mmol) of D-lactic acid, and the solution was stirred at 70°C. To the reaction mixture was added a small amount of D-lactic acid salt of R-nipecotamide, which was prepared in advance, as a seed crystal, and a diastereomer salt was precipitated. The reaction mixture was stirred for 1 hour at the same temperature, and then was cooled to 10°C while stirring for 6 hours. The mixture was further stirred for 10 hours. The precipitated diastereomer salt was filtered and collected. The diastereomer salt was washed with a mixed solvent of 10 mL of 1-butanol and 10 mL of ethyl acetate at approximately 5°C. After drying, the obtained white diastereomer salt weighed 7.27 g. The diastereomer salt was D-lactic acid salt of R-nipecotamide, and the yield was 42.7 %. The optical purity of R-nipecotamide obtained from the diastereomer salt was analyzed and was found to be 98.0 % ee.
¹H-NMR(DMSO-d₆, 400MHz) δppm: 7.47 (1H, s), 6.95 (1H, s), 3.71 (1H, q, J=13.7, 6.8Hz), 3.14-3.02 (2H, m), 2.80-2.64 (2H, m), 2.54-2.44 (1H, m), 1.90-1.84 (1H, m), 1.74-1.67 (1H, m), 1.62-1.43 (2H, m), 1.14 (3H, d, J=6.8Hz).

### Example 2 Optical Resolution of Nipecotamide

Two grams (2g, 15.6 mmol) of nipecotamide was dissolved in 10 mL of 1-butanol. To the solution was added 1.76 g ([α]_{D}²⁰ = -13° (c = 2.5, 1.5 mol/L NaOH), 85 % aqueous solution, 16.6 mmol) of L-lactic acid, and the solution was stirred at 50°C. To the reaction mixture was added a small amount of L-lactic acid salt of S-nipecotamide, which was prepared in advance, as a seed crystal, and a diastereomer salt was precipitated. The reaction mixture was stirred for 1 hour at 60°C, then was left to cool to room temperature while stirring, and was further stirred at the same temperature overnight. The precipitated diastereomer salt was filtered and collected, and was washed with 5 mL of 1-butanol. After drying, the obtained white diastereomer salt weighed 1.25 g. The diastereomer salt was L-lactic acid salt of S-nipecotamide, and the yield was 36.7 %. The optical purity of S-nipecotamide obtained from the diastereomer salt was analyzed and was found to be 99.0 % ee. ¹H-NMR(DMSO-d₆, 400MHz) δppm: 7.47 (1H, s), 6.95 (1H, s), 3.71 (1H, q, J=13.7, 6.8Hz), 3.14-3.02 (2H, m), 2.80-2.64 (2H, m), 2.54-2.44 (1H, m), 1.90-1.84 (1H, m), 1.74-1.67 (1H, m), 1.62-1.43 (2H, m), 1.14 (3H, d, J=6.8Hz).

### Example 3 Optical Resolution of Nipecotamide

The same operation as that shown in Example 2 was conducted except that the same amount of 1-butanol/ethyl acetate (1/1 (volume/volume)) was used instead of 1-butanol in Example 2, to obtain 1.40 g of white crystals of L-lactic acid salt of S-nipecotamide. The yield was 41.1 %. The optical purity of S-nipecotamide obtained from the diastereomer salt was 99.0 % ee.

### Example 4 Optical Resolution of Nipecotamide

The same operation as that shown in Example 2 was conducted except that the same amount of 1-butanol/methyl isobutyl ketone (1/1 (volume/volume)) was used instead of 1-butanol in Example 2, to obtain 1.40 g of white crystals of L-lactic acid salt of S-nipecotamide. The yield was 41.1 %. The optical purity of S-nipecotamide obtained from the diastereomer salt was 97.9 % ee.

### Example 5 Production of R-nipecotamide

Three grams (3g, 13.7 mmol) of D-lactic acid salt of R-nipecotamide obtained in Example 1 was mixed with 10 mL of water and 20 mL of 1-butanol. 1.74 g (16.4 mmol) of sodium carbonate was added and stirred while keeping the resulting mixture at 20 to 25°C. The mixture was left still and liquid separated, and an organic layer was collected. The aqueous layer was extracted with 20 mL of 1-butanol. The organic layer obtained and the organic layer previously obtained were combined and was washed with 5 mL of saturated salt water. The organic layer obtained was condensed, then 20 mL of 1-butanol was added, and the insoluble substance was filtered. By condensing the solution obtained, 0.90 g of R-nipecotamide in white crystals was obtained. The yield was 51 %. The optical purity of R-nipecotamide was analyzed and found to be 98.0 % ee.

### INDUSTRIAL APPLICABILITY

The production method of the present invention can provide an optically active nipecotamide with a simple operation and with efficiency, and is suitable for production on a commercial basis. By selecting D-form or L-form as the optically active lactic acid used, both R-nipecotamide and L-nipecotamide can be produced. Accordingly, the production method of the present invention is a very useful production method.

## Claims

1. A method for producing an optically active nipecotamide, the method comprising: a step of reacting nipecotamide with an optically active lactic acid in a solvent to prepare a diastereomer salt mixture and then allowing one of the diastereomer salts contained in the diastereomer salt mixture to precipitate; a step of collecting the precipitated diastereomer salt; and, a step of treating the collected diastereomer salt with a base to liberate an optically active nipecotamide.

2. The production method according to claim 1, wherein the solvent is one or more solvents selected from an alcohol, a ketone and an ester.

3. The production method according to claim 1, wherein the solvent is 1-butanol, a mixed solvent of 1-butanol and methyl isobutyl ketone, or a mixed solvent of 1-butanol and ethyl acetate.

4. The production method according to claim 1, wherein the solvent is a mixed solvent of 1-butanol and methyl isobutyl ketone.

5. The production method according to claim 1, wherein the solvent is a mixed solvent of 1-butanol and ethyl acetate.

6. The production method according to claim 1, comprising a step of reacting nipecotamide with an optically active lactic acid to prepare a diastereomer salt mixture and then allowing one of the diastereomer salts contained in the diastereomer salt mixture to precipitate, by adding an aqueous solution of an optically active lactic acid to nipecotamide dissolved in one or more solvents selected from an alcohol, a ketone and an ester.

7. The production method according to claim 6, wherein the solvent is 1-butanol, a mixed solvent of 1-butanol and methyl isobutyl ketone, or a mixed solvent of 1-butanol and ethyl acetate.

8. A diastereomer salt mixture of nipecotamide and an optically active lactic acid.

9. A diastereomer salt of an optically active nipecotamide and an optically active lactic acid.

10. A D-lactic acid salt of R-nipecotamide.

11. An L-lactic acid salt of S-nipecotamide.
